# EUROPÄISCHE PATENTANMELDUNG

(11) **EP 0 567 979 A2**
(43) Veröffentlichungstag der Anmeldung: **03.11.1993**
(21) Anmeldenummer: 93106760.7
(22) Anmeldetag: 27.04.1993
(51) Int. Cl.: A61M 5/32, A61M 25/06

(54) **Schutzkappe**

(30) Priorität: 29.04.1992 DE 4213844
(71) Anmelder: SÜDDEUTSCHE FEINMECHANIK GMBH, D-63607 Wächtersbach (DE)
(72) Erfinder: Link, Alfons, W-6465 Biebergemünd 2 (DE)
(74) Vertreter: Stoffregen, Hans-Herbert, Dr. Dipl.-Phys.

(57) **Zusammenfassung**

Es wird eine Schutzkappe (24) für eine eine Handhabe (14) aufweisende Kanüle (10) insbesondere fuhr die Gefäßpunktion wie extrakorporale Blutbehandlung vorgeschlagen, die einerseits problemlos entfernbar und andererseits ein quasi zwangsläufiges Abdecken der Kanüle nach deren Gebrauch ermöglicht. Hierzu kann die Schutzkappe mit einem Längsschlitz versehen sein, wobei zumindest ein Rand (32, 34) von diesem in einer in Längsrichtung der Handhabe verlaufenden Einbuchtung (28, 30) gefuhrt aufgenommen ist.

## Beschreibung

Die Erfindung bezieht sich auf eine Schutzkappe führ eine Kanüle insbesondere für die Gefäßpunktion wie extrakorporale Blutbehandlung wie Dialyse oder Plasmapherese mit gegebenenfalls einer die Kanüle aufnehmenden Kanülenhalterung, einer flügelartige Elemente aufweisenden Handhabe sowie mit einem aufschiebbaren Schlauch, wobei zum zumindest bereichsweise freiliegenden Kanülenhalter die Schutzkappe in Richtung der Handhabe verschiebbar ist und gegebenenfalls zumindest einen Längsschlitz aufweist, der von der Handhabe und/oder dem Kanülenhalter durchsetzbar ist.

Eine entsprechende Schutzkappe weist zwei Längsschlitze auf, die von Flügeln einer Handhabe durchsetzbar sind. Die Schlitze erstrecken sich von der Spitze der Schutzkappe bis in den Bereich des proximalen Endes, das die Kanülenhalterung bzw. ein auf diese geschobener Schlauch vollständig umgibt. Wird die Kanüle nicht benutzt, so ist die Schutzkappe derart auf die Kanülenhalterung geschoben, daß die angeschrägte Spitze der Kanüle abgedeckt ist. Folglich ist eine unbeabsichtigte Verletzung ausgeschlossen.

Bei einer Benutzung der Kanüle wird die Schutzkappe entlang des Schlauches gezogen, um sodann freigelegt zu sein. Nachteilig bei einer diesbezüglichen Handhabung ist, daß die Schutzkappe gegebenenfalls in einem Umfang entlang des Schlauches verrutschen kann, daß ein erneutes Bedecken der Kanüle mit der Schutzkappe umständlich ist, so daß nach wie vor eine Verletzungsgefahr nach einer Benutzung der Kanüle besteht. Eine solche ist jedoch insbesondere unter dem Gesichtspunkt von AIDS oder Hepatitis auszuschließen.

Zu erwähnen ist noch, daß die Schutzkappe nur solage festgelegt ist, wie diese den proximalen Endbereich der Kanülenhalterung umgibt, dies jedoch nur eine kurze Strecke ist, erfolgt ein häufig unerwünschtes Abrutschen der Schutzkappe und Bewegen dieser entlang des Schlauches.

Bekannte Kanülen mit Schutzkappen sind den DE 37 01 585 C2, SU 16 90 779 A1, SU 16 48 485, US 47 23 955, SU 15 72 648 A1 und SU 8 54 400 zu entnehmen.

Der vorliegenden Erfindung liegt das Problem zugrunde, eine Schutzkappe der zuvor beschriebenen Art so weiterzubilden, daß nicht nur ein problemloses Entfernen der Schutzkappe von der Kanüle selbst möglich ist, sondern daß sich ein Abdecken der Kanüle nach deren Gebrauch zwangsläufig ergibt, ohne daß ein umständliches Verschieben entlang eines Schlauches erforderlich wird.

Das Problem wird erfindungsgemäß einerseits dadurch gelöst, daß zumindest ein Rand des Längsschlitzes der Schutzkappe in einer in Längsrichtung der Handhabe verlaufenden Einbuchtung geführt aufgenommen ist. Vorzugsweise erfolgt jedoch eine Führung beider Ränder der Schutzkappe in entsprechende Längseinbuchtungen der Handhabe, die zwischen einem zylinderförmigen Basiskörper und den von diesem ausgehenden flügelartigen Elementen verläuft.

Durch diese konstruktiven Maßnahmen ist quasi über die gesamte Länge der Schutzkappe eine Führung entlang der Handhabe möglich, so daß ein kontrolliertes Entfernen von der Kanüle und ein sicheres Abdecken dieser nach deren Gebrauch möglich ist, wobei die Schutzkappe selbst auch bei der Benutzung der Kanüle im Bereich der Handhabe verharrt. Folglich ist die Schutzkappe insbesondere auch im entfernten Zustand griffbereit, so daß ein Benutzer zwangsläufig ein Abdecken der Kanüle vornehmen wird.

Auch wenn die Schutzkappe fortwährend mit der Handhabe in Verbindung bleibt, wird hierdurch deren Benutzung nicht behindert, da die abragenden Flügelelemente weiterhin frei zugänglich sind.

Durch den erfindungsgemäßen Aufbau der Schutzkappe ergibt sich im Querschnitt eine U-Form mit wulstartigen und einander zugewandten Rändern, also quasi eine einseitig offene Schutzkappe, die jedoch eine sichere Abdeckung der Kanüle selbst gewährleistet.

Eine entsprechende Schutzkappe ist nicht nur einfach herstellbar, sondern auch problemlos von der Spitze her auf die Handhabe schiebbar.

Damit die Schutzkappe eindeutige Positionen zu der Kanüle einnimmt, ist nach einem Vorschlag der Erfindung vorgesehen, daß die Schutzkappe sowohl im proximalen als im distalen Endbereich auf der Kanülenhalterung bzw. dem Basiskörper der Handhabe verrastbar ist.

Nach einem weiteren Vorschlag, bei dem eine entsprechende Verrastung nicht notwendigerweise erforderlich ist, geht die Schutzkappe von einer Schlauchklemme aus, so daß eine Lagefixierung der Schutzkappe von der Fixierung der Klemme am Schlauch selbst abhängig ist. Da die Schlauchklemme grundsätzlich in unmittelbarer Nähe der Handhabe auf den Schlauch einwirkt, ist insoweit gleichfalls sichergestellt, daß die Kanüle im erforderlichen Umfang abgedeckt bzw. freigelegt ist.

Ein selbständiger Lösungsvorschlag der Erfindung sieht vor, daß die Schutzkappe zwei an und für sich bekannte Schlitze aufweist, die jedoch im Abstand zum vorderen Ende der Schutzkappe enden und von den flügelartigen Elementen durchsetzbar sind. Auch mit dieser Konstruktion ist -abweichend vom Stand der Technik- ein problemloses Aufschieben der Schutzkappe auf die Kanüle von deren Spitze her möglich. Da der Schlitz jedoch im Abstand zum vorderen Ende endet, ist hierdurch der zusätzliche Vorteil gegeben, daß die Schutzkappe nur bis zu den flügelartigen Elementen der Handhabe von der Kanüle entfernbar ist, so daß infolgedessen die Schutzkappe gleichfalls stets griffbereit ist. In diesem Fall ist ein Verrasten der Schutzkappe nur in ihrem proximalen Bereich auf der Kanülenhalterung bzw. dem Basiskörper der Handhabe erforderlich.

Nach einem weiteren selbständigen Lösungsvorschlag ist die Schutzkappe in Längsrichtung der Kanüle zusammenschiebbar. Dabei kann die Schutzkappe zieharmonika-artig oder balgartig ausgebildet sein. Hierdurch ergibt sich der Vorteil, daß dann, wenn die Schutzkappe nach deren Zurückziehen losgelassen wird, quasi automatisch die Kanüle selbst wieder umgibt, da aufgrund der Eigenvorspannung des Schutzkappenmaterials dieses bestrebt ist, in die ursprüngliche Form zu gelangen.

Bei einer entsprechenden Konstruktion kann die Schutzkappe mit der die Kanüle umgebenden Kanülenhalterung bzw. der Handhabe fest verbundenen und vorzugsweise als integraler Bestandteil dieser ausgebildet sein.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung eines der Zeichnung zu entnehmenden Ausführungsbeispiels.

Es zeigen:
- Fig. 1: einen Längsschnitt durch eine erste Ausführungsform einer von einer Schutzkappe umgebenen Kanüle,
- Fig. 2: einen Schnitt entlang der Linien A-B in Fig. 1,
- Fig. 3: einen Längsschnitt durch eine zweite Ausführungsform einer von einer Schutzkappe umgebenen Kanüle,
- Fig. 4: einen Schnitt entlang der Linien C-D in Fig. 3,
- Fig. 5: einen Längsschnitt durch eine dritte Ausführungsform einer von einer Schutzkappe umgebenen Kanüle,
- Fig. 6: eine vierte Ausführungsform einer von einer Schutzkappe umgebenen Kanüle und
- Fig. 7: einen Schnitt entlang der Linien E-F in Fig. 6.

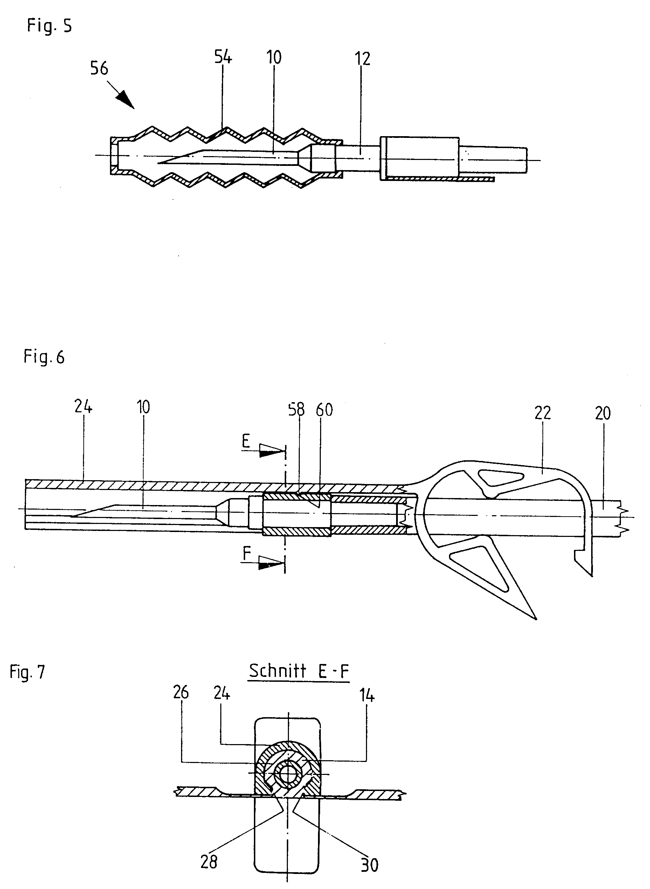

In den Figuren, in denen für gleiche Elemente gleiche Bezugszeichen benutzt werden, sind vom Aufbau hinreichend bekannte Kanülen insbesondere für die Gefäßpunktion wie extrakorporale Blutbehandlung wie Dialyse oder Plasmapherese dargestellt, die jeweils eine schräg angeschliffenen Kanüle (10), eine diese aufnehmende Kanülenhalterung (12) sowie eine Handhabe (14) umfaßt, von der zur Benutzung Flügel (16) und (18) ausgehen. Auf das kanülenspitzenabgewandte, also proximale Ende der Kanülenhalterung (12) ist ein Schlauch (20) aufschiebbar, der über eine in Fig. 6 rein beispielhaft dargestellten Klemme (22) abklemmbar ist.

Um eine Gefährdung durch die Kanülenspitze auszuschließen, insbesondere dann, wenn diese nach ihrer Benutzung herumliegt, werden die Kanülen von Schutzkappen umgeben, die nach der Benutzung der Kanüle (10) diese problemlos wieder abdecken können.

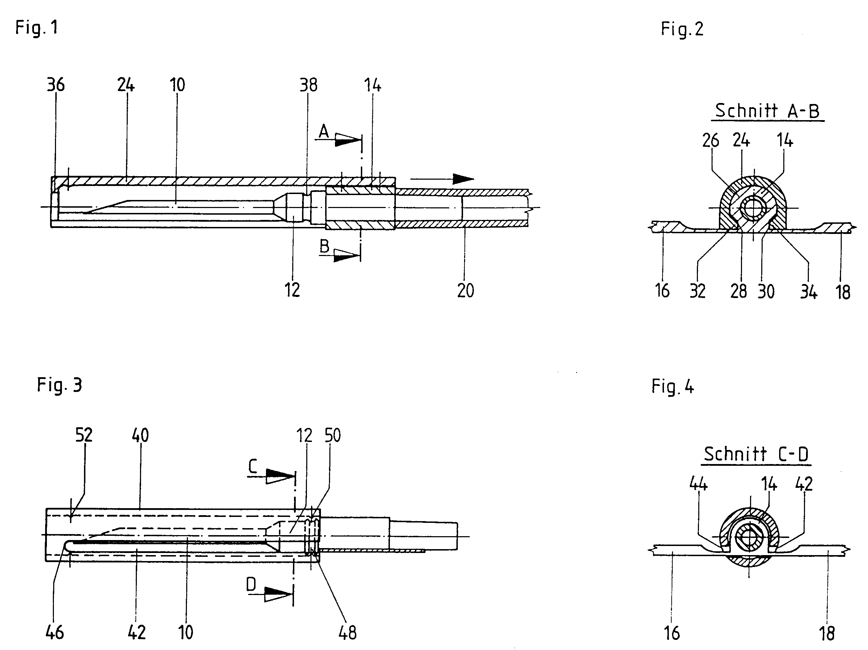

So ist nach den Ausführungsbeispielen der Fig. 1 und 2 eine Schutzkappe (24) vorgesehen, die aus einem einseitig offenen zylinderförmigen Körper (24) besteht, der in Längsrichtung der Kanüle (10) verschiebbar und dabei von einem zylinderförmigen Basiskörper (26) der Handhabe (14) geführt aufgenommen wird. Diese Führung erfolgt dadurch, daß zwischen dem Basiskörper (26) und den Flügelansätzen Längseinbuchtungen (28) und (30) ausgebildet sind, in die wulstartig ausgebildete Ränder (32) und (34) der Schutzkappe (24) eingreifen.

Mit anderen Worten weist der zylinderförmige und einseitig offene Körper der Schutzkappe (24) eine U-Form mit einander zugewandten wulstförmig ausgebidleten Rändern auf.

Um die Schutzkappe (24) nur soweit von der Kanüle (10) zu entfernen, daß diese zur Nutzung freigelegt ist, kann die Schutzkappe (24) an ihrem distalen Ende, und zwar in ihrem Stirnbereich auf der Kanülenhalterung (12) festgerastet werden. Hierzu weist die Kanülenhalterung (24) stirnseitig einen abgewinkelten und sich in Richtung der Kanülenlängsachse (10) verlaufenden Absatz (36) auf, der in eine Vertiefung (38) der Kanülenhalterung (12) einrastbar ist.

Das Ausführungsbeispiel der Fig. 3 und 4 zeigt eine Schutzkappe (40), die die Kanüle (10) mit der Kanülenhalterung (12) und der Handhabe (14) mit den Flügeln (16) und (18) im erforderlichen Umfang umgibt. Um die Schutzkappe (40) geführt entlang der Kanüle (10) zu verschieben, sind Längsschlitze (42) und (44) vorgesehen, die von den Flügeln (18) und (16) durchsetzbar sind. Dabei gehen die Längsschlitze (42) und (44) von der der Kanülenspitze abgewandten Stirnseite des Schutzkappenkörpers aus und enden im Abstand zum vorderen Ende. Hierdurch bedingt ist die Schutzkappe (40) nur soweit von der Kanüle (10) zurückziehbar, bis die der Kanülenspitze zugewandte Randbereich der Flügel (16) und (18) an dem Schlitzende (46) anstößt.

Um eine eindeutige Lagefixierung der Schutzkappe auf der Kanülenhalterung (12) und damit zur Kanüle (10) selbst zu ermöglichen, kann die Kanülenhalterung eine zur Verrastung dienende Vertiefung (48) aufweisen, in die im proximalen und gegebenenfalls auch im distalen Endbereich der Schutzkappe vorhandene Vorsprünge (50) bzw. (52) einbringbar sind.

Nach dem Ausführungsbeispiel der Fig. 5 wird die Kanüle (10) von einer balg-bzw. zieharmonikaartig ausgebildeten Schutzkappe (54) umgeben, die von der Kanülenhalterung (12) ausgeht und gegebenenfalls integraler Bestandteil dieser sein kann. Um die Kanüle (10) freizulegen, ist es nur erforderlich, daß die Schutzkappe (54) von ihrem vorderen Ende (56) her eine Krafteinwirkung in Richtung der Kanülenhalterung (12) erfährt, so daß hierdurch ein Zurückziehen der Schutzkappe (54) erfolgt. Soll die Kanüle (10) durch die Schutzkappe (54) abgedeckt werden, so braucht letztere nur losgelassen zu werden, so daß ein Zurückbewegen der Schutzkappe (54) in ihre ursprüngliche oder nahezu ursprüngliche Form aufgrund der Einspannung selbsttätig erfolgt.

Daß den Fig. 6 und 7 zu entnehmende Ausführungsbeispiel stellt eine Variante der Fig. 1 und 2 in bezug auf die Schutzkappe selbst dar. So umgibt die Schutzkappe (24) derart den Basiskörper (26) der Handhabe (14), daß eine Führung in den Längsausbuchtungen (28) und (30) erfolgt.

Mit anderen Worten ist die Schutzkappe (24) als einseitig offener hohlzylindrischer Körper ausgebildet.

Abweichend vom Vorschlag der Fig. 1 und 2 ist jedoch die Schutzkappe (24) mit der Klemme (22) verbunden, so daß die Lage der Klemme (22) gleichzeitig die Position der Schutzkappe (24) bestimmt. Auch auf diese Weise ist ein einfaches Freilegen bzw. Abdecken der Kanüle (10) möglich. Ergänzend kann die Schutzkappe (24) einen Rastvorsprung (58) aufweisen, der in eine entsprechend angepaßte Rastnut (60) im Basiskörper (26) eingreift.

## Patentansprüche

1. Schutzkappe für eine Kanüle insbesondere für die Gefäßpunktion wie extrakorporale Blutbehandlung wie Dialyse oder Plasmapherese mit gegebenenfalls einer die Kanüle aufnehmenden Kanülenhalterung, mit einer flügelartige Elemente aufweisenden Handhabe sowie mit einem aufschiebbaren Schlauch, wobei zum zumindest bereichsweise Freilegen der Kanüle die Schutzkappe in Richtung der Kanülenhalterung verschiebbar ist und gegebenenfalls zumindest einen Längsschlitz aufweist, der von der Handhabe oder Teilen dieser durchsetzbar ist,
**dadurch gekennzeichnet**,
daß zumindest ein Rand (32,34) des Längsschlitzes der Schutzkappe (24) in einer in Längsrichtung der Handhabe (14) verlaufenden Einbuchtung (28, 30) geführt aufgenommen ist.

2. Schutzkappe nach Anspruch 1,
**dadurch gekennzeichnet**,
daß die Handhabe (14) einen zylinderförmigen Basiskörper (26) mit den von diesem ausgehenden flügelartigen Elementen (16, 18) aufweist, wobei im Übergangsbereich zwischen dem Basiskörper und den flügelartigen Elementen Längseinbuchtungen (28,30) vorhanden sind, in denen sich die Ränder (32,34) des Längsschlitzes der Schutzkappe (24) erstrecken.

3. Schutzkappe nach Anspruch 1 oder 2,
**dadurch gekennzeichnet**,
daß die Schutzkappe (24) im Querschnitt eine U-Form mit wulstartigen und einander zugewandten Rändern aufweist.

4. Schutzkappe für eine Kanüle insbesondere für die Gefäßpunktion wie extrakorporale Blutbehandlung wie Dialyse oder Plasmapherese mit gegebenenfalls einer die Kanüle aufnehmenden Kanülenhalterung, mit einer flügelartige Elemente aufweisenden Handhabe sowie mit einem aufschiebbaren Schlauch, wobei zum zumindest bereichsweise Freilegen der Kanüle die Schutzkappe in Richtung der Kanülenhalterung verschiebbar ist und gegebenenfalls zumindest einen Längsschlitz aufweist, der von der Handhabe oder Teilen dieser durchsetzbar ist,
**dadurch gekennzeichnet**,
daß die Schutzkappe (40) zwei Schlitze (42, 44) aufweist, die im Abstand zum vorderen Ende der Schutzkappe enden und von den flügelartigen Elementen (16,18) der Handhabe (14) durchsetzbar sind.

5. Schutzkappe nach zumindest einem der vorhergehenden Ansprüche,
**dadurch gekennzeichnet**,
daß die Schutzkappe (24) mit einer den Schlauch abklemmenden Klemme (22) verbunden oder integraler Bestandteil dieser ist.

6. Schutzkappe nach zumindest einem der vorhergehenden Ansprüche,
**dadurch gekennzeichnet**,
daß die Schutzkappe (24,40) im proximalen und/oder distalen Endbereich auf der Kanülenhalterung (12) oder der Handhabe (14) verrastbar ist.

7. Schutzkappe für eine Kanüle insbesondere für die Gefäßpunktion wie extrakorporale Blutbehandlung wie Dialyse oder Plasmapherese mit gegebenenfalls einer die Kanüle aufnehmenden Kanülenhalterung, mit einer flügelartige Elemente aufweisenden Handhabe sowie mit einem aufschiebbaren Schlauch, wobei zum zumindest bereichsweise Freilegen der Kanüle die Schutzkappe in Richtung der Kanülenhalterung verschiebbar ist,
**dadurch gekennzeichnet**,
daß die Schutzkappe (54) in Längsrichtung der Kanüle (10) zusammenschiebbar ist.

8. Schutzkappe nach Anspruch 7,
**dadurch gekennzeichnet**,
daß die Schutzkappe (54) zieharmonika- oder balgartig ausgebildet ist.

9. Schutzkappe nach Anspruch 7 oder 8,
**dadurch gekennzeichnet**,
daß die Schutzkappe (54) mit der Kanülenhalterung (12) oder der Handhabe verbunden und gegebenenfalls als integraler Teil mit dieser ausgebildet ist.
